(19) Europäisches Patentamt
European Patent Office
Office européen des brevets

(11) **EP 3 895 702 B1**

(12) **EUROPEAN PATENT SPECIFICATION**

(45) Date of publication and mention of the grant of the patent:
**24.09.2025 Bulletin 2025/39**

(21) Application number: **21168796.7**

(22) Date of filing: **16.04.2021**

(51) International Patent Classification (IPC):
***A61K 31/19** (2006.01)* ***A61K 45/06** (2006.01)*
***A61P 35/00** (2006.01)*

(52) Cooperative Patent Classification (CPC):
(C-Sets available)
**A61K 31/19; A61K 45/06; A61P 35/00** (Cont.)

(54) **DERIVATIVE OF VALPROIC ACID AND DICHLOROACETATE USED FOR THE TREATMENT OF CANCER**

DERIVAT VON VALPROINSÄURE UND DICHLORACETAT ZUR BEHANDLUNG VON KREBS

DÉRIVÉ D'ACIDE VALPROÏQUE ET DE DICHLORACÉTATE UTILISÉ POUR LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.04.2020 LT 2020521**

(43) Date of publication of application:
**20.10.2021 Bulletin 2021/42**

(73) Proprietor: **Lietuvos sveikatos mokslu universitetas**
**44307 Kaunas (LT)**

(72) Inventors:
- **Stakisaitis, Donatas**
  **44307 Kaunas (LT)**
- **Valanciute, Angelija**
  **44307 Kaunas (LT)**
- **Balnyte, Ingrida**
  **44307 Kaunas (LT)**
- **Lesauskaite , Vaiva**
  **44307 Kaunas (LT)**
- **Jukneviciene, Milda**
  **44307 Kaunas (LT)**
- **Staneviciute, Jurate**
  **44307 Kaunas (LT)**
- **Jasaitis, Darius Juozas**
  **08317 Vilnius (LT)**

(74) Representative: **Zaboliene, Reda**
**Metida**
**Business center Vertas**
**Gyneju str. 16**
**01109 Vilnius (LT)**

(56) References cited:
**WO-A1-2005/105055** **WO-A1-2006/108276**
**US-A1- 2015 231 176**

- **STANEVICIUTE J. ET AL: "PO-426 Gender disparity in lung adenocarcinoma susceptibility in experimental mice model", ESMO OPEN : CANCER HORIZONS, vol. 3, 1 June 2018 (2018-06-01), London, pages A190, XP055832818, ISSN: 2059-7029, DOI: 10.1136/esmoopen-2018-EACR25.452**
- **STANEVICIUTE JURATE ET AL: "The effect of dichloroacetate on male rat thymus and on thymocyte cell cycle", INTERNATIONAL JOURNAL OF IMMUNOPATHOLOGY AND PHARMACOLOGY, vol. 29, no. 4, 1 December 2016 (2016-12-01), pages 818 - 822, XP055832830, ISSN: 2058-7384, DOI: 10.1177/0394632016674019**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/19, A61K 2300/00**

## Description

FIELD OF THE INVENTION

The invention relates to the use of a combination of valproic acid (VPA) and dichloroacetate (DCA) salts (VPA-DCA) in the treatment of glioma.

BACKGROUND OF THE INVENTION

Chemotherapy with combinations of anticancer medicinal products is used to obtain an additional or synergistic anticancer effect. Rational combinations of medicines have different mechanisms of action that inhibit tumor growth. In this way, the emergence of resistance to treatment is be delayed, the doses of drugs used for treatment and their toxicity and the risk of side effects are reduced. The application of the synergism of different classes of anticancer drug combinations is based on their known mechanisms of pharmacological action, i. e. the synergistic effect of combinations of alkylating agents and purine analogues (cyclophosphamide and fludarabine), platinum preparations and antimetabolites (combinations of cisplatin and fluorouracil; and cisplatin and gemcitabine) and combinations of two antimetabolites (combination of gemcitabine and capecitabine) were investigated. The research of new combinations of medicines for the treatment of cancer is an important area of oncology science.

U.S. Pat. No. 9,556,113 describes the use of a combination of dichloroacetate and oxamate and combinations of dichloroacetate and synthesized oxamate prodrugs in the treatment of cancer of various localization. The patent characterizes the anticancer efficacy and the mechanisms of the pharmacological action of a combination of two medicines that complement each other, i. e. act synergistically. These medicinal products can be used as primary or adjunctive therapy (separately or in combination) and in parallel with systemic chemotherapy.

EP1708692B1 describes the use of DCA to improve cardiac contractile function, coronary blood flow after ischemia-reperfusion. Combination with DCA allows reducing the doses of inotropic agents (dopamine, epinephrine, ephedrine, phenylephrine, dobutamine) as cardiac index improves. DCA amount of 50 mg/kg is administered by intravenous infusion to neonates, children, adults; when used in combination with inotropic agents protects the myocardium from damage. DCA improves myocardial function recovery and metabolism after ischemic episodes (during heart surgery, hemorrhagic shock, hypoxia).

U.S. Pat. No. 8,607,724 describes the use of DCA and chemical equivalents thereof in the treatment of cancer to induce apoptosis or reduce resistance to apoptosis. Amount of sodium DCA of 10 to 100 mg/kg is injected in combination with other pro-apoptotic agents or chemotherapy for the treatment of non-small cell lung, breast cancer and glioblastoma.

U.S. Pat. No. 20,140,066,482 and EP2026787 disclose the treatment of non-insulin-dependent diabetes in combination with the authorized agents repaglinide and metformin. Repaglinide promotes the release of insulin from pancreatic ß-cells, while metformin reduces insulin resistance, inhibits hepatic glucose production, and increases muscle and adipose tissue sensitivity to insulin. The compounds of the combination differ in their chemical formula, pharmacokinetics, and mechanisms of pharmacological action. The patented combination product produces a synergistic effect of the two components in the treatment of non-insulin-dependent diabetes.

It is disclosed the use of a combination of sodium dichloracetate (DCA) and sodium valproate (NaVP) on urethane induced lung tumours in mice (Staneviciute J. et al.: "PO-426 Gender disparity on lung adenocarcinoma susceptibility in experimental mice model", ESMO OPEN: Cancer Horizons, vol. 3, 1 June 2018, page A190, WP055832818, London). The combination is administered not simultaneously, but alternatively. However, VPA-DCA combination provided in the inventon is intended to use for different kind of cancer.

It is disclosed the effect of the combination of DCA with NaVP on thymocyte proliferation in tissue (Staneviciute J. et al.: "The effect of dichloroacetate on male rat thymus and on thymocyte cell cycle", International Journal of immunopathology and Pharmacology, vol. 29, No. 4, 1 December 2016, pages 818-822, XP055832830). The study described in said document is related with effect of DCA on the thymus as well as on the thymocyte cycle regulation in male rats. The combination is administered not simultaneously, but alternatively. VPA-DCA combination provided in the invention provides synergy in anticancer action, including metabolic modulation and defines specific medical use of VPA-DCA combination as anticancer therapy.

WO2006/1082276 discloses the use of DCA or salts thereof for treating non-small cell lung cancer, glioblastoma and breast carcinoma.

WO2005/105055 discloses the use of VPA for the treatment of breat cancer, estrogen receptor-independent breats cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarin cancer, astrocytomas, gliomas, skin cancer, melanoma, head and neck cancer, small cell lung carcinoma, non-small cell lung carcinoma, leukemias, lymphomas and/or other blood cell cancer.

The specific effect obtained for combination of DCA and VPA on glioma is provided by present invention.

## BRIEF DESCRIPTION OF THE INVENTION

It is an object of the invention to provide a novel combination of active substances that comprises an effective amount of (a) dichloroacetate (DCA) or a pharmaceutically acceptable salt thereof, and (b) valproic acid (VPA) or a pharmaceutically acceptable salt thereof. In one embodiment, the pharmaceutically acceptable salt of dichloroacetate is sodium dichloroacetate or magnesium dichloroacetate. The effect of a beneficial combination of VPA-DCA magnesium salts is important for improving the balance of $Mg^{2+}$ ions in the body. $Mg^{2+}$ ions are important in inhibiting tumorogenesis.

In another embodiment of the present invention, the pharmaceutically acceptable salt of valproic acid is sodium valproate or magnesium valproate.

The present invention provides a pharmaceutical composition comprising a combination of active substances in association with pharmaceutically acceptable excipients.

The VPA-DCA combination is intended for use as medicine, specifically for the treatment of glioma. This combination is characterized by a new pharmacological mechanism of action of DCA and VPA: both agents inhibited cellular Na-K-2Cl co-transporter (NKCC1) in rat thymocytes and renal tubules (NKCC2); NKCC1 is important for the development of tumorogenesis associated with increased tumor cell NKCC1 expression and co-transporter's activity; said pharmacological effect of VPA and DCA reduces $Na^+$ and $Cl^-$ in tumor cells and extracellular environment. The decreased intracellular concentration of $Cl^-$ anion is associated with decreased proliferation of tumor cells and increased apoptosis. Said combination reduces serum glucose, inhibits aerobic glycolysis, inhibits the tumor cell proliferation *in vivo* and *in vitro.*

Preclinical studies indicate that DCA and VPA when co-administered with radiotherapy and/or chemotherapy, delay the development of resistance to chemotherapy or radiotherapy. Our experimental studies suggest that VPA-DCA combination therapy provides a synergistic effect of the anticancer components of the investigational medicinal product, inhibits T cell proliferation, induces apoptosis, inhibits tumor growth *in vivo,* which makes to reduce DCA dose and the risk of adverse events.

The combination of active substances is intended for use in the treatment of glioma . Said combination is used for the treatment of cancer with concomitant hyperglycemia associated with metabolic syndrome.

A combination of active substances comprising dichloroacetate (DCA) or a pharmaceutically acceptable salt thereof and (b) valproic acid (VPA) or a pharmaceutically acceptable salt thereof is administered as a combined preparation or administered simultaneously or sequentially as separate medicinal products. The treatment of cancer with said combination of active substances is used in preclinical and clinical trials.

## BRIEF DESCRIPTION OF THE DRAWINGS

Figure 1. Serum glucose level of control and VPA-DCA-treated rats

Figure 2. 24-hour diuresis in control and VPA-DCA-treated rats

Figure 3. 24-hour daily urinary ions excretion of control and VPA-DCA-treated rats after 28 days of treatment

Figure 4. Expression of NKCC1 co-transporter RNA in thymocytes from control and VPA-DCA-treated male rats

Figure 5. Effects of DCA and VPA-DCA combination on glioblastoma (GB) tumor transplanted on chicken chorioallantoic membrane

Figure 6. Mechanisms of the pharmacological action of DCA and VPA, the anticancer synergistic effect of VPA-DCA combination (scheme)

Figure 7. Proliferating cell nuclear antigen (PCNA) expression in GB tumor transplanted on chicken chorioallantoic membrane treated with a combination of VPA-DCA (4 mM VPA-5 mM NaDCA or 2 mM VPA-3 mM MgDCA; 7 days treatment duration) *in vivo*

Figure 8. Testosterone concentration (nM/l) data in blood serum of control and VPA-NaDCA-treated male rats (n = 6 group) after 28 days dosage (long horizontal lines represents median, short horizontal lines - minimum and maximum values). Rats were treated with 150 mg/kg/d VPA and 100 mg/kg/d NaDCA combination dose

DETAILED DESCRIPTION OF THE INVENTION

VPA and DCA are investigational medicinal products that have anticancer effects. VPA-DCA is an important adjunct to anticancer treatment regimens. DCA inhibits pyruvate dehydrogenase (PDH) kinases in cells of various tumors; VPA is an inhibitor of histone deacetylases (HDACs).

The Figures 2 and 8 and embodiments including VPA-DCA effect on diuresis and the rat blood serum testosterone concentration are not according to the invention and are present for illustration purposes only.

***The use of VPA in the treatment of cancer.*** VPA preparations are used in treating neurological diseases: therapeutic indication for the treatment of epilepsy was registered 6 decades ago. Preclinical and clinical studies on the anticancer effects of VPA are currently underway. VPA is an epigenetic regulator of carcinogenesis. VPA induces epigenetic inhibition of HDAC class Ia (HDAC1 and HDAC2), class Ib (HDAC3), class Ic (HDAC8), and class IIa (HDAC4, HDAC5, and HDAC7), increases acetylation of nucleosome-forming histones H2, H3, and H4, condensation of chromatin, that modifies the mechanisms of oncogene silencing, activation of cancer-inhibiting transcription factors associated with apoptosis, cell differentiation, cell cycle, and inhibition of angiogenesis. Elevated expression of HDAC is an indicator of cancer malignancy. VPA inhibits the proliferation of tumor cells, promotes their apoptosis, inhibits neoangiogenesis; reduces the expression of GB cell nuclear proliferation antigen associated with GB tumor growth, cell apoptosis *in vivo.* The anticancer effects of VPA in the cell are related to the production of reactive oxygen species, leading to apoptosis of GB cells and autophagy. VPA is a modulator of the immune response and may mediate immune-induced tumor cell death. Treatment of GB with VPA and temozolomide demonstrated adaptive immune responses *in vivo* and *in vitro*; VPA inhibits NKCC1 RNA expression in rat thymocytes. Preclinical studies have shown that VPA inhibits the development of glioblastoma, neuroblastoma, breast, ovarian, cervical, colorectal, prostate, thyroid, liver cancers, melanoma, Ackin, and ovarian tumors.

VPA increases susceptibility to renal cancer chemotherapy with various medicines; the sorafenib-VPA combination synergistically inhibits the viability of liver cancer cells, promotes apoptosis. VPA significantly increases the apoptosis of ovarian cancer cells, sensitivity to cisplatin. Co-administration of valproic acid with rapamycin or temozolomide increases the autophagic effect. VPA-bevacizumab combination with gemcitabine and docetaxel suppresses resistance to chemotherapy. The impact of VPA is exacerbated by concomitant use of irinotecan or etoposide; the combination of VPA and etoposide has a higher cytotoxic effect on GB cells and a higher sensitivity of GB tumor to chemotherapy; synergistic antiproliferative or increased sensitivity of GB tumor was observed when VPA was co-administered with mitoxantrone, etoposide. VPA in combination with temozolomide or taxol strengthens the apoptotic effect on GB cells, inhibiting the activity of the multidrug resistance protein.

VPA is an anticancer medicine used in combination with radiotherapy: VPA induced the susceptibility of primary GB cells to radiotherapy, with a decrease in tumor cell survival and increased $\gamma$-radiation-induced cytotoxicity. VPA increased the susceptibility of temozolomide treatment to radiation therapy in GB cells that were resistant to temozolomide, which was associated with an increase in cell death by apoptosis and autophagy, and with an increase in cell number of the cell cycle G2 or G2-M.

***DCA** anticancer effect.* DCA is an anticancer investigational medicinal product that has been shown to be effective in preclinical and clinical trials. DCA as a regulator of mitochondrial metabolism in cancer cells causes changes in the metabolism of cancer cells by altering their glycolytic phenotype. The main DCA pharmacological mechanisms are:

(1) DCA inhibits pyruvate dehydrogenase (PDH) kinases and thus maintains the active pyruvate dehydrogenase complex (PDHC), promotes the metabolism of pyruvate and glucose in mitochondria. The expression of PDH kinase isoforms in various tumor cells is increased; PDH kinases are cancer therapy target. Metabolism of tumors of various localization is characterized by decreased oxidative phosphorylation in mitochondria and more active aerobic glycolysis, a phenomenon known as the Warburg effect, which results in increased resistance of tumor cells to apoptosis associated with increased lactic acid production in tumor tissue. DCA treatment reduces lactic acid synthesis in tumor cells and the intercellular environment.

(2) Treatment with DCA promotes the generation of reactive oxygen species that triggers a variety of pro-apoptotic changes in tumor cells.

(3) Our experimental studies revealed a new mechanism of action of DCA. DCA induced a diuretic effect associated with inhibition of Na-K-2Cl co-transporter (NKCC2) in the kidney of rats: increased $Na^+$, $Cl^-$ and $K^+$ as well as $Mg^{2+}$ and $Ca^{2+}$ ions urinary excretion and inhibition of NKCC1 RNA expression in thymocytes. NKCC1 is a marker of cancer and a target of tumor progression; it regulates cell proliferation, apoptosis, tumor invasion; increased NKCC1 activity is associated with cell cycle progression, cell volume. NKCC activity is inhibited by oxidative stress (effect of reactive radicals).

(4) Preclinical studies have shown synergistic effects of DCA combinations with other authorized anticancer agents or with radiotherapy, delaying resistance to chemotherapy, promoting apoptosis of cancer cells, or increasing or decreasing the cytotoxicity of anticancer drugs.

DCA promotes mitochondrial function and inhibits the conversion of pyruvate to lactic acid and alanine, inhibits the development of lactic acid acidosis (lactic acidosis) in the tumor microenvironment. Lactate is a mediator of inflammation; increased concentration in the tumor is accompanied by the accelerated release of IL-17A by T lymphocytes and macrophages, chronic activation of inflammation. Lactate inhibits monocyte migration and cytokine release, induces vascular endothelial growth factor production, IL-8 expression in pancreatic, ovarian cancer tissue, stimulates TNF-$\alpha$ production. DCA inhibits the development of acquired lactic acidosis, which is associated with tumor recurrence, metastasis, and poorer cancer prognosis. Lactic acidosis has been associated with resistance to radiotherapy, chemotherapy; it reduces the efficacy of doxorubicin, mitoxantrone, and daunorubicin. Women are more prone to lactic acidosis; estrogens stimulate, and testosterone inhibits lactate production.

One of the factors in solid tumor resistance to therapy is tumor tissue hypoxia. DCA treatment inhibits hypoxia-induced factor (HIF-1) gene reproduction in the cell. HIF-1 activity is directly related to PDH kinaze K1 (PDHK1) expression and lactic acidosis.

DCA treatment results in increased infiltration of T lymphocytes into the tumor, which induces a stronger immune activity directed to the tumor, which may also be associated with a decrease in lactate content in the tumor and its microenvironment; increased lactate concentration inhibits T lymphocyte function. Our studies of rat thymus have shown that DCA affects T lymphocyte proliferation, NKCC1 expression in T lymphocytes, and this effect is gender-dependent and gonad hormones-dependent.

The anticancer effect of DCA is manifested by inhibition of neoangiogenesis.

Preclinical studies have shown that DCA has a synergistic effect when used in combination with other anticancer agents to modify oxidative stress, neoangiogenesis, immune response; DCA-arginase combination has stronger inhibition of breast cancer growth *in vivo*; bevacizumab-DCA combination significantly inhibits growth and neoangiogenesis of glioma stem cell tumors transplanted into female mice; DCA-doxorubicin combination may be important in improving breast cancer chemotherapy strategy; head and neck tumors with PDHK2 overexpression are resistant to cisplatin; DCA significantly reduces their resistance to cisplatin *in vitro* and *in vivo*; DCA-paclitaxel combination reduces paclitaxel-resistant lung cancer cell survival: the resistance of these cells to chemotherapy is associated with overexpression of PDK2, and adjunctive DCA therapy reduces resistance to paclitaxel; DCA-vemurafenib combination is highly potential in the treatment of melanoma; DCA reduces the resistance of hepatocellular carcinoma to sorafenib in male mice; DCA-capecitabine combination enhances the anticancer effect to mice B16 melanoma and human lung cancer xenografts transplanted into male mice; DCA-paclitaxel combination supresses the development resistance to paclitaxel in male mice lung adenocarcinoma; DCA-bortezomib combination significantly increases survival of mice with multiple myeloma xenografts.

Application of radiotherapy in combination with DCA in a model of female mice glioblastoma tumor has shown that the effect of DCA is associated with enhanced sensitivity to radiotherapy, leading to a clear increase of cell number in cell cycle G2-M, and also increase the survival of female mice with GB. DCA as an adjunct medicament could be promising in the treatment of cancer resistant to radiotherapy and chemotherapy.

The dose used in DCA anticancer clinical trials ranged from 10 to 17 mg/kg/day orally for long-term treatment of glioblastoma, small cell lung cancer, melanoma, and colon cancer; said doses of DCA did not impair quality of life. Phase I-III clinical trials of congenital lactic acidosis with DCA have shown that oral DCA is a safe treatment for children with no evidence of renal, pulmonary, bone marrow, or cardiac toxicity. The most common adverse reaction of DCA is mild peripheral neuropathy. Administration of VPA in addition to chemotherapy inhibits the development of peripheral neuropathy.

**Justification of the synergistic anticancer effect of VPA-DCA**

***Interaction of DCA and VPA salts combination characterization in solution or dry mixtures*** Sodium dichloroacetate is a highly water-soluble salt, it is a strong electrolyte and is completely ionized in solution. This is a salt of strong acid ($Cl_2HCOOH$) and strong base (NaOH), thus hydrolysis does not occur in an aqueous solution. The solution medium is practically neutral, there is no chemical interaction between the salt ions. The chemical formula and structure of the dichloroacetate and valproic acid salts are given in Table 1 below.

Table 1

| Material | | Formula | Structural formula |
|---|---|---|---|
| COMBINATION | Sodium dichloroacetate | $Cl_2CHCOONa$ | 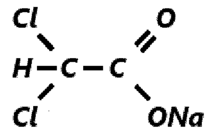 |
| | Sodium valproate | $(C_3H_7)_2CHCOONa$ | 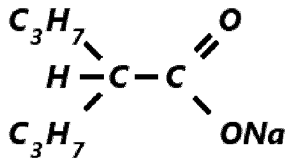 |
| | | | |
| COMBINATION | Magnesium dichloroacetate | $(Cl_2CHCOO)_2Mg$ | 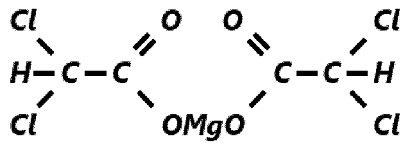 |
| | Magnesium valproate | $((C_3H_7)_2CHCOO)_2Mg$ | 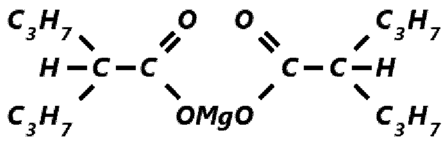 |

To characterize the state of ions in a solution, the so-called effective (conditional) ion concentrations $C_{ef}$ is calculated:
$c_{ef} = f \times c$, where f is the dimensionless ion activity coefficient; in dilute solutions f depends on the ionic charge z and the ionic force I of the solution:
$lgf = -0.5 \times z^2 \times I^{0.5}$. If the concentration of the solution, e.g., 0.1 mol/l, then

$$I = 0.1 \text{ and } lgf(\text{for } Na^+ \text{ and } Cl_2HCOO^- \text{ ions}) = -0.5 \times (1)^2 \times 0.1^{0.5} = -0.158,$$

$$f = 10^{-0.158} = 0.7; \text{ then } c_{ef}(Na^+) = c_{ef}(Cl_2HCOO^-) = 0.7 \times 0.1 = 0.07 \text{ mol/l.}$$

Theoretical calculations of the ionic activities of the solution show that there is no interaction between the combinations of salts, thus the formulation does not affect the biological activity of the anions, which depends only on the number per unit volume thereof, i.e., on concentration thereof in the medium.

The above statements are also applicable to an aqueous solution of sodium valproate which also undergoes hydrolysis, thus forming an aqueous solution of a strong base (NaOH) and a weak valproic acid ($(C_3H_7)_2CHOOH$) (Table 1). The name of valproic acid according to systemic nomenclature is 2-propyl-pentanoic acid, the anion thereof hydrolyzes:

$$(C_3H_7)_2CHCOO^-(aq) + H_2O(s) \leftrightarrow (C_3H_7)_2CHCOOH(aq) + OH^- \quad (aq)$$

The medium becomes slightly basic. The ionization equilibrium constant of this acid is $K_r \times 2.2 \cdot 10^{-5}$, hence the equilibrium constant of the hydrolysis reaction is $K_h = K_{H2O}/K_r = 1 \cdot 10^{-14}/2.5 \cdot 10^{-5} = 4 \cdot 10^{-10}$. Hence the degree of hydrolysis of the acid ion is $h = (K_h/c)^{0.5} = (4 \cdot 10^{-10}/0.1)^{0.5} = 6.3 \cdot 10^{-5}$ or 0.006%. Therefore, the decrease in anion concentration due to hydrolysis is negligible, and its biological activity does not change at all. The solution of the sodium dichloroacetate and sodium valproate, is a homogeneous mixture of ions, with almost no chemical interaction in the combination, like in the individual salt solutions. The biological activity of anions in the mixture is not interfered by any chemical interaction of ions,

their activity depends only on their amount, i.e., on the concentration.

Any solid phase interionic conversion takes place at room temperature in the combinations of the two dry crushed salts mentioned above, which are mechanical mixtures of cations and anions. The biological properties of such mechanical mixtures depend only on the ratio of mixed salt content. This indicates that a pharmaceutical formulation of these salt combinations is possible.

The situation is similar to the use of aqueous solutions and solid mixtures of corresponding magnesium salts, which are less soluble than sodium salts. The processes of hydrolysis of salt ions are slightly more manifest only in the solutions of magnesium salts, which are derivatives of weak bases and weak acids. The calculations show that the impact of the hydrolysis on the initial ion concentrations is negligible and does not substantially alter the existing anion concentrations, and thus their biological activity. Pharmaceutical formulations in the form of sodium and magnesium valproate solutions and salts are authorized.

***Synergistic effect of VPA-DCA in inhibiting aerobic glycolysis.*** Both components of the combination inhibit aerobic glycolysis (Warburg effect). Studies have shown that VPA suppressed aerobic glycolysis in neuroblastoma cells by inhibiting glucose uptake into the cell, reducing the production of lactate and ATP. The VPA inhibits aerobic glycolysis by reducing the amount of E2F transcription factor 1 (E2F1), which inhibits the expression of glucose-6-phosphate isomerase and phosphoglycerate kinase 1 (PGK1). DCA suppresses aerobic glycolysis by inhibiting pyruvate dehydrogenase kinases and thus sustaining an active pyruvate dehydrogenase complex; VPA-DCA promotes mitochondrial glucose metabolism in tumor cells due to these mechanisms.

VPA-DCA reduces glucose levels in blood serum. Following the treatment (aqueous solution of VPA-DCA orally for 28 days) serum glucose was statistically significantly reduced in male rats: the control group had 6.37 (3.90 to 8.81) mM/l, the VPA-DCA combination group had 3.13 (2.17-3.78) mM/l. Figure 1 shows the serum glucose level of control and VPA-DCA-treated rats. The data (mM/l) are presented as medians (horizontal long line), with minimum or maximum values (small horizontal dashes). Thus, VPA-DCA has a direct effect on serum glucose level and tumor progression. One of the strategies for treating tumors is lowering serum glucose concentration.

***Synergistic effect of VPA-DCA on diurnal diuresis associated with inhibition* of *Na-K-2Cl cotransporter*.** Long-term treatment (for 28 days) of male rats with VPA-DCA (aqueous solution orally) resulted in a significant increase in daily diuresis per 100 g of rat weight; in comparison with control, it was 3.95 (3.2-5.74) ml and 2.83 (0.95-4.3) ml, respectively. Figure 2 depicts 24 hours diuresis of control and VPA-DCA-treated rats. Diuresis is expressed in ml/100 g of rat weight: median (horizontal long line) and minimum and maximum values (small horizontal dashes).

***Synergistic effect of VPA and DCA on $Na^+$ and $Cl^-$ ion homeostasis.*** Our preclinical studies have shown for the first time that VPA and DCA inhibit the NKCC2 co-transporter in rat kidney. The increase in 28-day diuresis in male rats treated for 28 days with VPA-DCA is associated with a synergistic effect of VPA and DCA on the excretion of $Na^+$, $K^+$ and $Cl^-$ ions in the diurnal urine. Diurnal urinary $Na^+$ excretion was 0.278 (0.095-0.390) mM/100 g of rat weight in the control group, and 0.376 (0.30-0.530) mM in VPA-DCA-treated rats; $K^+$ excretion was 0.374 (0.319-0.471), and 0.465 (0.388-0.638) mM/100 g of rat weight, respectively; there was also a clear increase in diurnal urinary chloride: 0.181 (0.063-0.246) mM/100 g of rat weight in the control group and 0.242 (0.198-0.34) mM/100 g of rat weight in the VPA-DCA-treated group. Figure 3 depicts the 24-hour ion excretion in diurnal urine of control and VPA-DCA-treated rats after 28 days of treatment. Data are presented in mM/100 g of rat weight as median (horizontal long line) and minimum without maximum values (small horizontal dashes).

Increased concentration of sodium chloride (NaCl) in the medium is associated with the Warburg effect, the development of lactic acidosis in tumor cells, and more active cell proliferation *in vitro*; NaCl mediates the proangiogenic effect of the tumor. A rich NaCl diet and increased NaCl concentrations in extracellular medium induce inflammatory cascades associated with carcinogenesis.

***Synergistic effect of VPA and DCA on NKCC1 RNA expression in rat thymocytes. A*** statistically significantly higher ΔCT value was found in the group of male VPA-DCA-treated rats in comparison with control (6.174 and 5.106, respectively) by analyzing the changes of the expression in rat thymocytes after VPA-DCA treatment. Expression of the co-transporter coding gene decreased after treatment with a combination of 52% compared to the control group. Figure 4 shows the expression of NKCC1 co-transporter RNA in thymocytes from control and VPA-DCA-treated male rats. A - ΔCT value obtained by normalizing the expression of the NKCC1 (*Slc12a2* gene) with the expression of the *Gapdh* gene (median (horizontal long line), and the maximum and minimum values (small horizontal dashes)); B - changes in NKCC1 expression determined by use of $2^{-\Delta\Delta CT}$ method; $^a p = 0.041$, compared to the control.

Preclinical studies indicate that NKCC inhibitors reduce intracellular $Cl^-$ level ($[Cl^-]i$); increased $[Cl^-]i$ is important for tumorogenesis, cell proliferation, tumor progression. VPA, DCA inhibits rat thymocyte proliferation, a cell cycle *in vivo.*

***Synergistic effect of VPA and DCA on glioblastoma tumor (GB) graft on chicken embryo chorioallantoic membrane (CAM).*** Figure 5 shows the effect of DCA and VPA-DCA on formed tumors of the glioblastoma U87 cell line on CAM. It was found that 90.9% of cases in the control group have been invasive into GB mesenchyme (A-a and B). Invasion into the CAM mesenchyme was detected in 50% of cases with 10 mM NaDCA treatment of GB (A-b and B). Invasion rate reaches 14.3% in GB treatment with 5 mM MgDCA (B). The incidence of invasion into the CAM mesenchyme was 9.1% in

the group treated with the combination of 4 mM VPA and 5 mM DCA (A-c and B). Differences in angiogenesis between the control GB group and VPA-DCA-treated tumors were observed on the fifth day after GB tumor transplantation following intravascular administration of fluorescent dextran solution i.e., GB group tumor shows a "spindle circle" of blood vessels formed around the tumor; the number of vessels attracted in the GB group treated with the combination of 4 mM VPA and 5 mM DCA was significantly reduced, and the tumor radiance was weaker than in the control group (C-b).

Figure 5 shows the effect of DCA and VPA-DCA combination on GB transplanted tumor on chicken chorioallantoic membrane. A - morphological changes of the chorioallantoic membrane in the groups studied: a - tumor formed from glioblastoma U87 MG cells; GB injured the integrity of the chorionic epithelium (ChE) (indicated by arrows) and invaded the CAM mesenchyme; b - GB treated with 10 mM NaDCA; no invasion of the CAM mesenchyme was detected, ChE injury already started (location marked with an arrow); c - GB treated with a combination of 4 mM VPA and 5 mM DCA; GB formed on the CAM surface, ChE integrity damaged (location marked with an arrow), CAM thinned (scale - 200 μm). B - incidence of GB tumor invasion into the CAM mesenchyme in the groups studied: control - untreated glioblastoma U87 MG tumors (n = 11), treated with 10 mM sodium DCA (GB-10 mM NaDCA; n = 14), treated with 5 mM magnesium DCA (GB-5 mM MgDCA; n = 7), and treatment with 5 mM NaDCA and 2 mM sodium VPA combination (GB-VPA-DCA, n = 11). C - fluorescence biomicroscopy of angiogenesis on the 5th day after tumor transplantation on CAM: a - untreated GB tumors, b - GB-4 mM VPA-5 mM DCA group; VPA-DCA treated tumor fluorescence is weaker compared to the control group tumor; a and b-a short arrow indicates the blood vessels attracted by the tumor, long arrow indicates a tumor (scale - 1 mm).

Figure 6 shows the mechanisms of the pharmacological action of DCA and valproic acid, anticancer synergistic effect scheme of VPA-DCA combination.

Figure 7 shows proliferating cell nuclear antigen (PCNA) expression in GB tumor transplanted on chicken chorioallantoic membrane treated with a combination of VPA-DCA (4 mM VPA-5 mM NaDCA or 2 mM VPA-3 mM MgDCA; 7 days treatment duration) *in vivo.*

A. Histological expression of PCNA-positive cells in GB-control tumor and tumor treated with VPA-DCA combination. Dark brown nuclei indicate PCNA-positive cells.

B. The graph shows the median (long horizontal dash) and min-max values (short horizontal dash) of PCNA-positive cells in each group. Compared to GB-control (n = 9), treatment with 4 mM VPA-5 mM NaDCA (n = 7) or 2 mM VPA-3 mM MgDCA (n = 7) significantly reduced the number of PCNA-positive cells in GB tumor tissue.

VPA-DCA is a potential new anticancer medicament. VPA-DCA may allow to reduce the DCA dose and to reduce the risk of adverse events of the components of the product. Adjunctive therapy with VPA-DCA would improve the anticancer therapeutic efficacy.

Treatment with NaDCA lowers serum testosterone, follicle-stimulating hormone (FSH) and luteinizing hormone (LH) levels in blood serum of male rats. Our study shows that treatment with VPA-DCA combination significantly reduces serum testosterone levels in male rats (Fig. 8).

## Claims

1. A combination comprising an effective amount of (a) dichloroacetate (DCA) or a pharmaceutically acceptable salt thereof and (b) valproic acid (VPA) or a pharmaceutically acceptable salt thereof for use in the treatment of glioma.

2. The combination for use in the treatment of glioma according to claim 1, **characterized in that** (a) the pharmaceutically acceptable salt of dichloroacetate is sodium dichloroacetate or magnesium dichloroacetate.

3. The combination for use in the treatment of glioma according to claim 1, **characterized in that** (b) the pharmaceutically acceptable salt of valproic acid is sodium valproate or magnesium valproate.

4. The combination for use in the treatment of glioma according to any one of claims 1 to 3 together with pharmaceutically acceptable excipients.

5. The combination for use in the treatment of glioma according to any one of claims 1 to 4, **characterized in that** the dichloroacetate (DCA) or a pharmaceutically acceptable salt thereof and (b) valproic acid (VPA) or a pharmaceutically acceptable salt thereof are administered as combined formulation or are administered simultaneously as individual medicinal products.

6. The combination for use in the treatment of glioma according to any one of claims 1 to 5, **characterized in that** it is

administered in combination with radiotherapy and/or chemotherapy.

## Patentansprüche

1. Kombination, umfassend eine wirksame Menge von (a) dichloroacetate (DCA) oder einem pharmazeutisch annehmbaren Salz davon und (b) valproic acid (VPA) oder einem pharmazeutisch annehmbaren Salz davon zur Verwendung bei der Behandlung von Gliom.

2. Kombination zur Verwendung bei der Behandlung von Gliom gemäß Anspruch 1, **dadurch gekennzeichnet, dass** das pharmazeutisch annehmbare Salz von DCA Natriumdichloroacetat oder Magnesiumdichloroacetat ist.

3. Kombination zur Verwendung bei der Behandlung von Gliom gemäß Anspruch 1, **dadurch gekennzeichnet, dass** das pharmazeutisch annehmbare Salz von VPA Natriumvalproat oder Magnesiumvalproat ist.

4. Kombination zur Verwendung bei der Behandlung von Gliom gemäß einem der Ansprüche 1 bis 3 zusammen mit pharmazeutisch annehmbaren Hilfsstoffen.

5. Kombination zur Verwendung bei der Behandlung von Gliom gemäß einem der Ansprüche 1 bis 4, **dadurch gekennzeichnet, dass** DCA oder ein pharmazeutisch annehmbares Salz davon und VPA oder ein pharmazeutisch annehmbares Salz davon als kombinierte Formulierung verabreicht oder gleichzeitig als einzelne Arzneimittel verabreicht werden.

6. Kombination zur Verwendung bei der Behandlung von Gliom gemäß einem der Ansprüche 1 bis 5, **dadurch gekennzeichnet, dass** sie in Kombination mit Strahlentherapie und/oder Chemotherapie verabreicht wird.

## Revendications

1. Association comprenant une quantité efficace de (a) dichloroacetate (DCA) ou d’un sel pharmaceutiquement acceptable de celui-ci et (b) valproic acid (VPA) ou d’un sel pharmaceutiquement acceptable de celui-ci pour une utilisation dans le traitement du gliome.

2. Association pour une utilisation dans le traitement du gliome selon la revendication 1, **caractérisée en ce que** le sel pharmaceutiquement acceptable du DCA est le dichloroacétate de sodium ou le dichloroacétate de magnésium.

3. Association pour une utilisation dans le traitement du gliome selon la revendication 1, **caractérisée en ce que** le sel pharmaceutiquement acceptable du VPA est le valproate de sodium ou le valproate de magnésium.

4. Association pour une utilisation dans le traitement du gliome selon l’une quelconque des revendications 1 à 3, conjointement avec des excipients pharmaceutiquement acceptables.

5. Association pour une utilisation dans le traitement du gliome selon l’une quelconque des revendications 1 à 4, **caractérisée en ce que** le DCA ou un sel pharmaceutiquement acceptable de celui-ci et le VPA ou un sel pharmaceutiquement acceptable de celui-ci sont administrés sous forme d’une formulation combinée ou administrés simultanément sous forme de médicaments distincts.

6. Association pour une utilisation dans le traitement du gliome selon l’une quelconque des revendications 1 à 5, **caractérisée en ce qu’**elle est administrée en combinaison avec une radiothérapie et/ou une chimiothérapie.

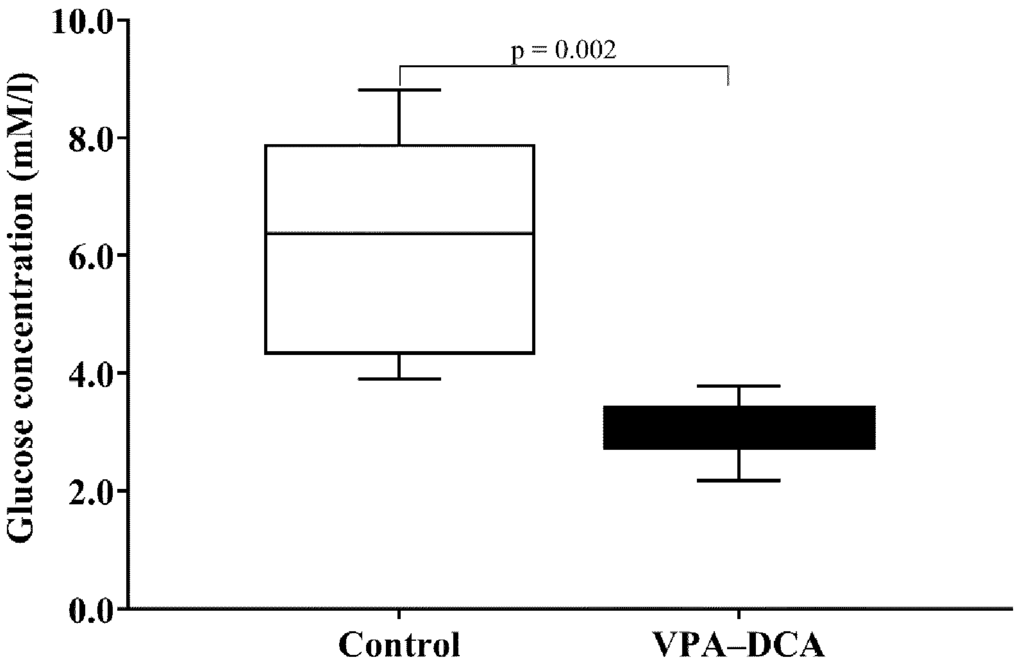
10.0
8.0
6.0
4.0
2.0
0.0
Glucose concentration (mM/l)
p = 0.002
Control
VPA–DCA

Fig. 1

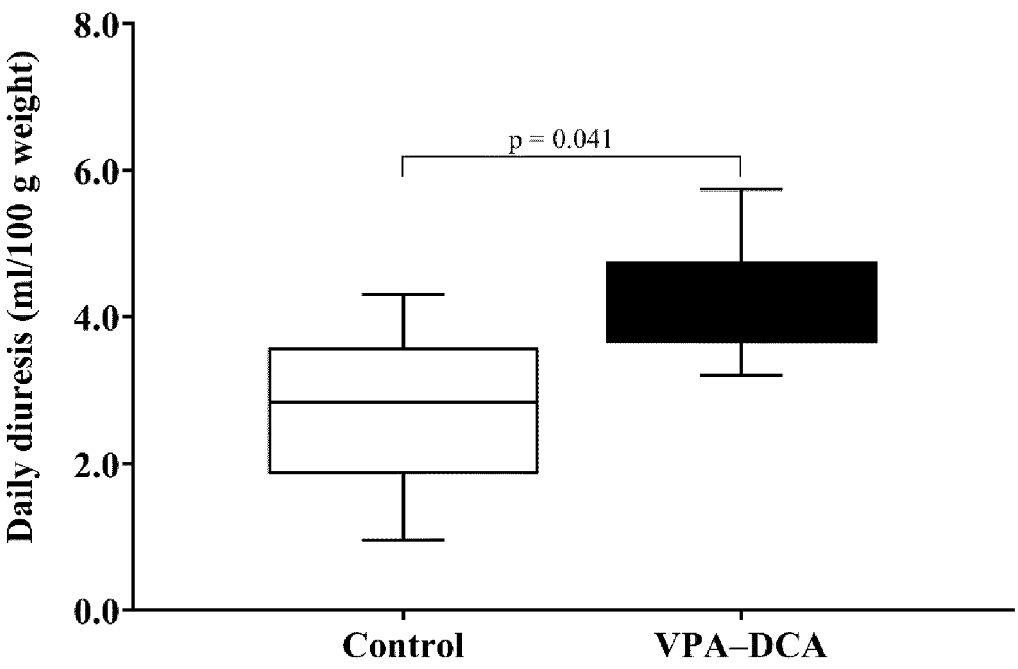
8.0
6.0
4.0
2.0
0.0
Daily diuresis (ml/100 g weight)
p = 0.041
Control
VPA–DCA

Fig. 2

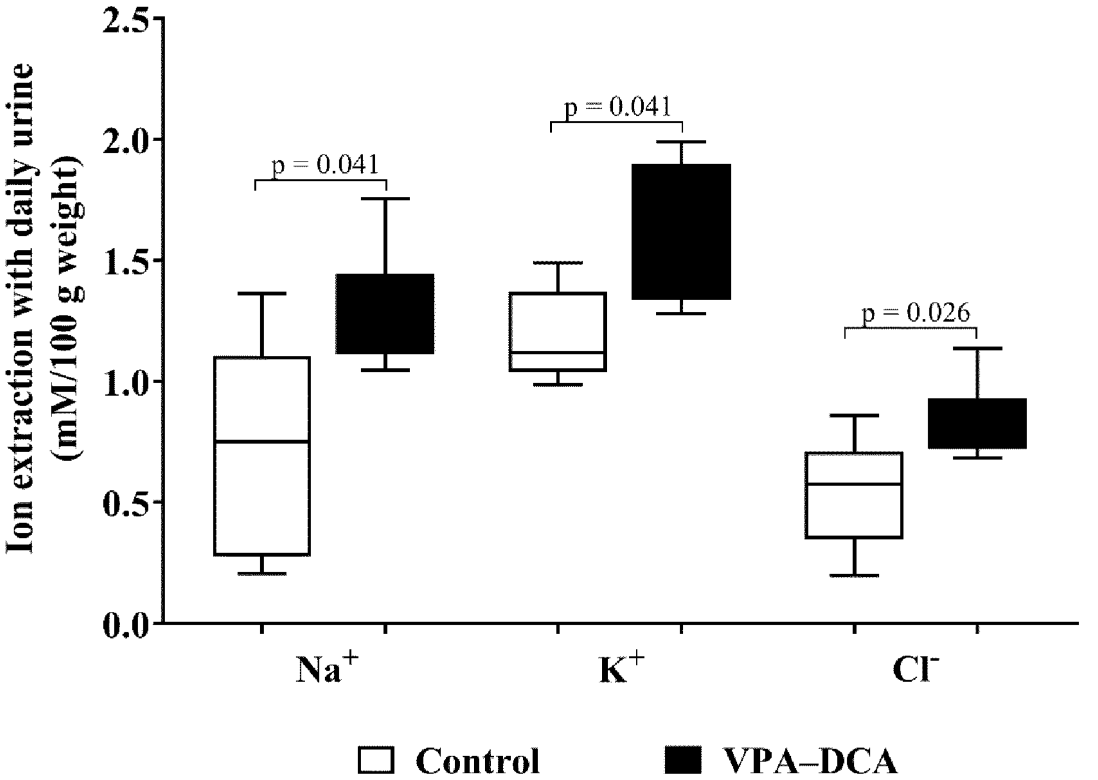
Ion extraction with daily urine (mM/100 g weight)
2.5
2.0
1.5
1.0
0.5
0.0
p = 0.041
p = 0.041
p = 0.026
Na+
K+
Cl-
Control
VPA–DCA

Fig. 3

A

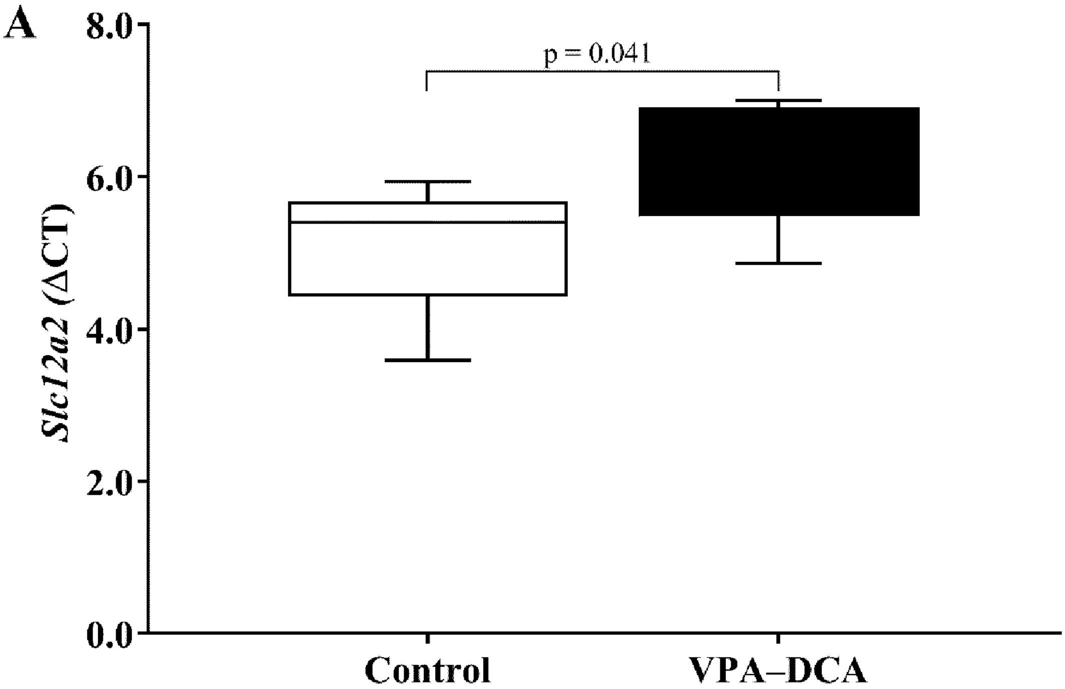


B

| Research group | CT average | | ΔCT | ΔΔCT | $2^{-\Delta\Delta CT}$ |
|---|---|---|---|---|---|
| | *Gapdh* | *Slc12a2* | | | |
| Control | 24.655 | 29.761 | 5.106 | 1.068 | 0.477 |
| VPA–DCA | 24.150 | 30.324 | 6.174[a] | | |

Fig. 4

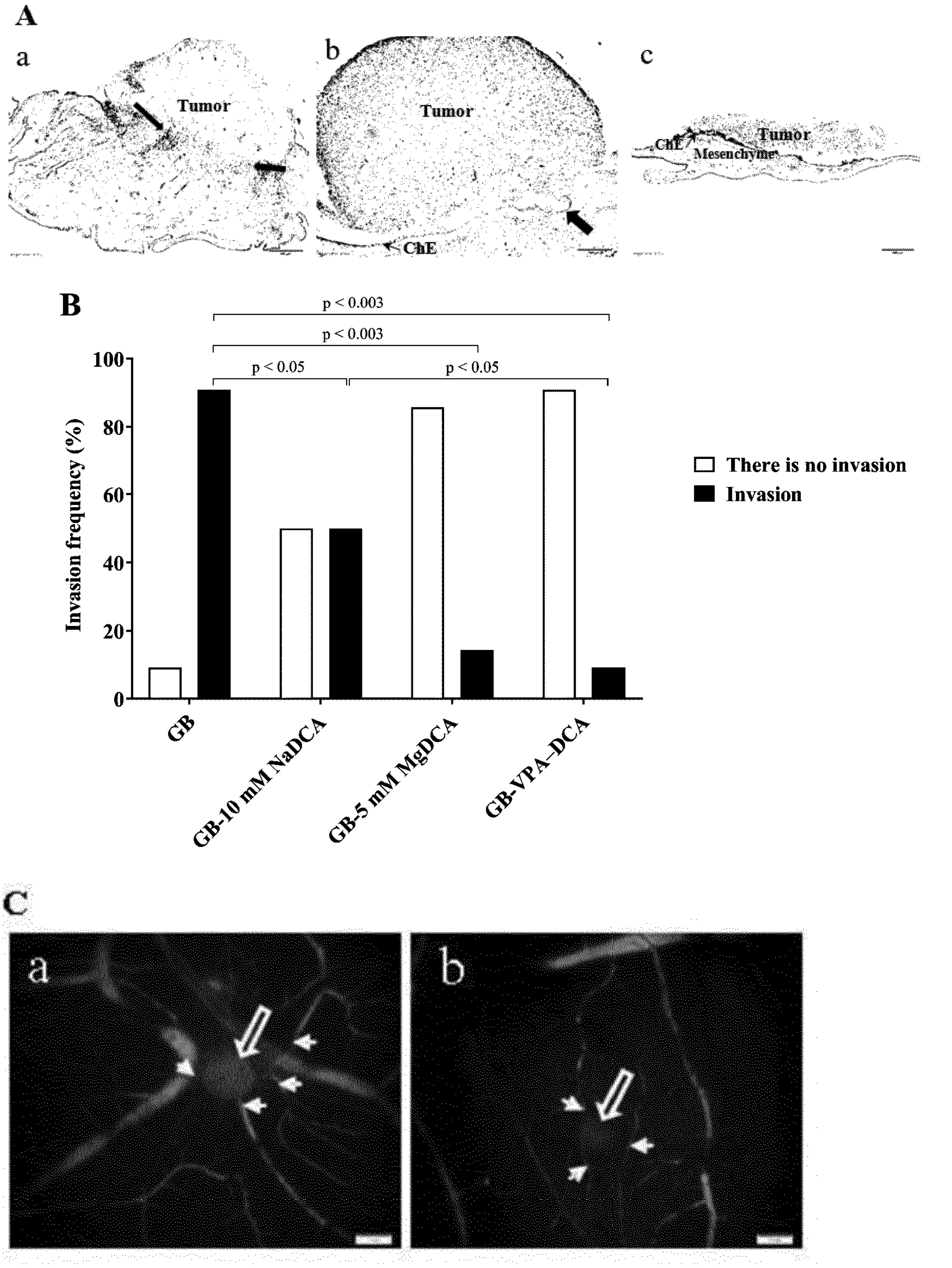
A
a
Tumor
b
Tumor
ChE
c
ChE
Tumor
Mesenchyme
B
p < 0.003
p < 0.003
p < 0.05
p < 0.05
Invasion frequency (%)
100
80
60
40
20
0
GB
GB-10 mM NaDCA
GB-5 mM MgDCA
GB-VPA–DCA
There is no invasion
Invasion
C
a
b

Fig. 5

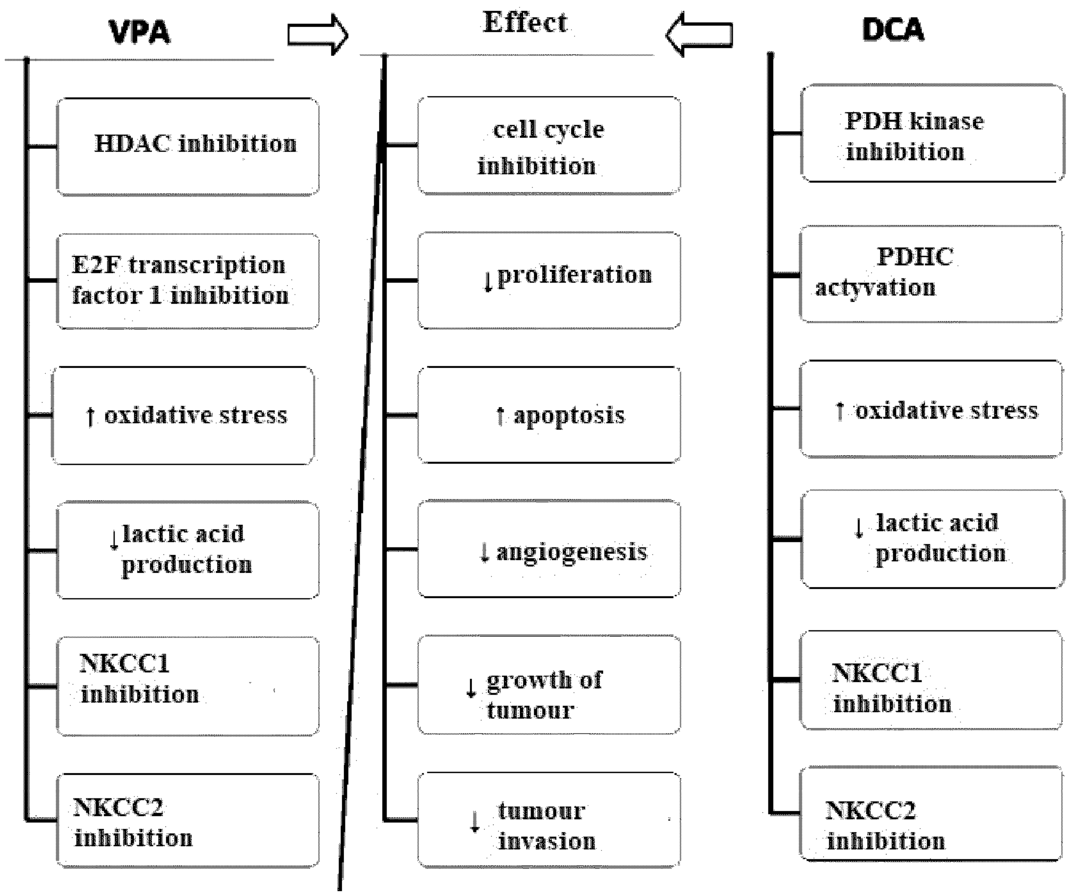
VPA
Effect
DCA
HDAC inhibition
E2F transcription factor 1 inhibition
↑ oxidative stress
↓ lactic acid production
NKCC1 inhibition
NKCC2 inhibition
cell cycle inhibition
↓ proliferation
↑ apoptosis
↓ angiogenesis
↓ growth of tumour
↓ tumour invasion
PDH kinase inhibition
PDHC actyvation
↑ oxidative stress
↓ lactic acid production
NKCC1 inhibition
NKCC2 inhibition

Fig. 6

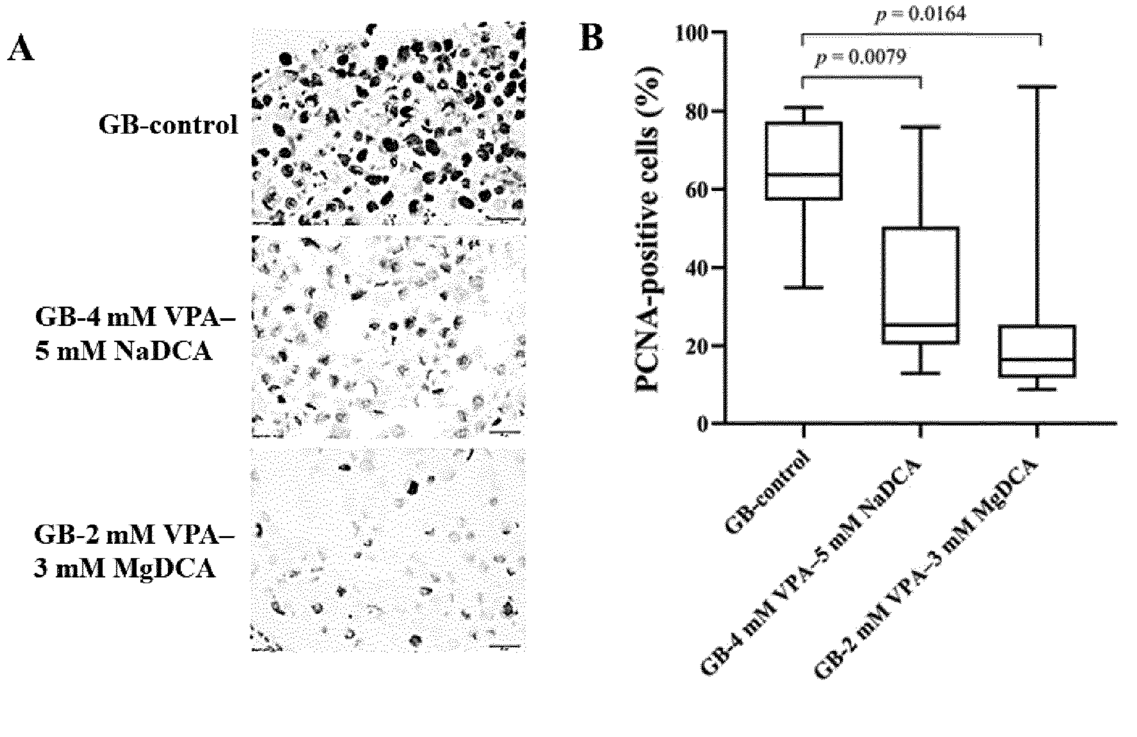
A
GB-control
GB-4 mM VPA–
5 mM NaDCA
GB-2 mM VPA–
3 mM MgDCA
B
PCNA-positive cells (%)
100
80
60
40
20
0
p = 0.0164
p = 0.0079
GB-control
GB-4 mM VPA–5 mM NaDCA
GB-2 mM VPA–3 mM MgDCA

Fig. 7

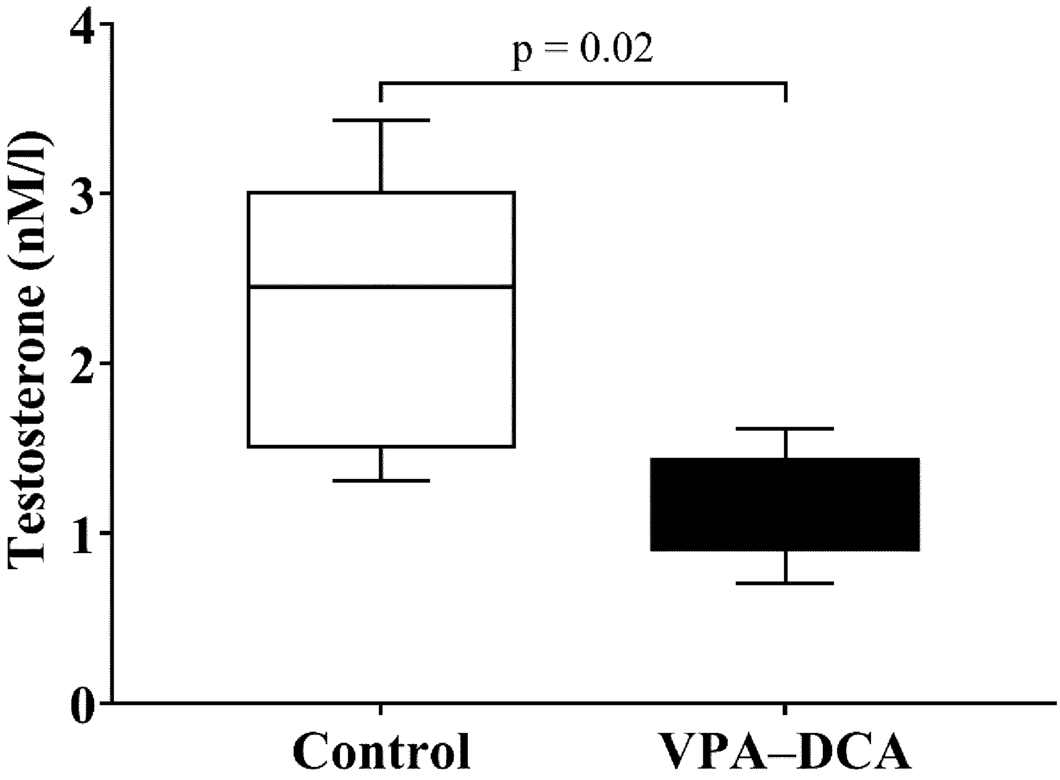


Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9556113 B
- EP 1708692 B1
- US 8607724 B
- US 20140066482 A
- EP 2026787 A
- WO 20061082276 A
- WO 2005105055 A


### Non-patent literature cited in the description


- **STANEVICIUTE J. et al.** PO-426 Gender disparity on lung adenocarcinoma susceptibility in experimental mice model. *ESMO OPEN: Cancer Horizons*, 01 June 2018, vol. 3, A190
- **STANEVICIUTE J. et al.** The effect of dichloroacetate on male rat thymus and on thymocyte cell cycle. *International Journal of immunopathology and Pharmacology*, 01 December 2016, vol. 29 (4), 818-822